(19) Europäisches Patentamt
European Patent Office
Office européen des brevets

(11) EP 3 257 868 A1

# (12) EUROPEAN PATENT APPLICATION
published in accordance with Art. 153(4) EPC

(43) Date of publication:
**20.12.2017 Bulletin 2017/51**

(21) Application number: **16749338.6**

(22) Date of filing: **12.02.2016**

(51) Int Cl.:
***C07K 17/08*** (2006.01) ***C07K 14/00*** (2006.01)
***C12N 7/08*** (2006.01) ***C12N 9/00*** (2006.01)
***C12N 15/00*** (2006.01)

(86) International application number:
**PCT/JP2016/054195**

(87) International publication number:
**WO 2016/129695 (18.08.2016 Gazette 2016/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **13.02.2015 JP 2015026489**

(71) Applicants:
- **National University Corporation Kyoto Institute of Technology Sakyo-ku Kyoto-shi, Kyoto 606-8585 (JP)**
- **Nissan Chemical Industries, Ltd. Chiyoda-ku Tokyo 101-0054 (JP)**

(72) Inventors:
- **KUMADA, Yoichi Kyoto-shi Kyoto 606-8585 (JP)**
- **OTSUKI, Ryoko Kyoto-shi Kyoto 606-8585 (JP)**
- **AKAI, Ryota Kyoto-shi Kyoto 606-8585 (JP)**
- **KATAYAMA, Junko Tokyo 101-0054 (JP)**
- **MATOBA, Kazutaka Funabashi-shi Chiba 274-8507 (JP)**

(74) Representative: **Patentanwälte Gierlich & Pischitzis Partnerschaft mbB Gerbermühlstraße 11 60594 Frankfurt am Main (DE)**

## (54) PEPTIDES HAVING AFFINITY FOR POLYDIMETHYLSILOXANE, AND USES THEREOF

(57) An object of the present invention is to provide a polydimethylsiloxane substrate to which a peptide having an affinity for polydimethylsiloxane (PDMS) is bound; a method for immobilizing a target protein on a polydimethylsiloxane substrate; a peptide having an affinity for PDMS; a polynucleotide encoding a peptide having an affinity for PDMS; and a vector using thereof. A polydimethylsiloxane substrate to which a peptide having an affinity for polydimethylsiloxane is bound, the peptide comprising the following peptide (1a) or (1b), or a fragment thereof: (1a) a peptide comprising an amino acid sequence represented by at least one member selected from the group consisting of SEQ ID NOs: 1 to 9; and (1b) a peptide comprising the amino acid sequence of (1a) in which one or more amino acids are deleted, substituted, and/or added, the peptide having an affinity for polydimethylsiloxane.

EP 3 257 868 A1

SEQUENCE LISTING

```
<110>  NATIONAL UNIVERSITY CORPORATION KYOTO INSTITUTE OF TECHNOLOGY
<110>  NISSAN CHEMICAL INDUSTRIES, LTD.

<120>  Peptide having affinity for Poly dimethylsiloxane

<130>  P15-186

<150>  JP 2015-026489
<151>  2015-02-13

<160>  18

<170>  PatentIn version 3.5

<210>  1
<211>  30
<212>  PRT
<213>  Escherichia coli

<400>  1

Met Val Met Pro Gly Asp Asn Ile Lys Met Val Val Thr Leu Ile His
1               5                   10                  15

Pro Ile Ala Met Asp Asp Gly Leu Arg Phe Ala Ile Arg Glu
            20                  25                  30


<210>  2
<211>  45
<212>  PRT
<213>  Escherichia coli

<400>  2

Val Gly Pro Asn Asn Val Pro Tyr Ile Val Ala Thr Ile Thr Ser Asn
1               5                   10                  15

Ser Ala Gly Gly Gln Pro Val Ser Leu Ala Asn Leu Lys Ala Met Tyr
            20                  25                  30

Ser Ile Ala Lys Lys Tyr Asp Ile Pro Val Val Met Asp
        35                  40                  45


<210>  3
<211>  31
<212>  PRT
<213>  Escherichia coli

<400>  3

Asn Asn Ser Trp Thr Arg Val Ala Phe Ala Gly Leu Lys Phe Gln Asp
1               5                   10                  15

Val Gly Ser Phe Asp Tyr Gly Arg Asn Tyr Gly Val Val Tyr Asp
            20                  25                  30
```

<210> 4
<211> 30
<212> PRT
<213> Escherichia coli

<400> 4

Leu Gly Trp Ser Gln Tyr His Asp Thr Gly Phe Ile Asn Asn Asn Gly
1 5 10 15

Pro Thr His Glu Asn Gln Leu Gly Ala Gly Ala Phe Gly Gly
20 25 30

<210> 5
<211> 20
<212> PRT
<213> Escherichia coli

<400> 5

Val Gly Pro Asn Asn Val Pro Tyr Ile Val Ala Thr Ile Thr Ser Asn
1 5 10 15

Ser Ala Gly Gly
20

<210> 6
<211> 17
<212> PRT
<213> Escherichia coli

<400> 6

Leu Ala Val Gly Leu Tyr Asp Gly Met Asn Leu Asp Trp Leu Ala Tyr
1 5 10 15

Arg

<210> 7
<211> 32
<212> PRT
<213> Escherichia coli

<400> 7

Asn Tyr Gly Val Val Tyr Asp Val Thr Ser Trp Thr Asp Val Leu Pro
1 5 10 15

Glu Phe Gly Gly Asp Thr Tyr Gly Ser Asp Asn Phe Met Gln Gln Arg
20 25 30

<210> 8

<211> 17
<212> PRT
<213> Escherichia coli

<400> 8

Phe Gly Gly Asp Thr Tyr Gly Ser Asp Asn Phe Met Gln Gln Arg Gly
1 5 10 15

Asn

<210> 9
<211> 29
<212> PRT
<213> Escherichia coli

<400> 9

Asp Ala Gly Ile Asn Thr Asp Asn Ile Val Ala Leu Gly Leu Val Tyr
1 5 10 15

Gln Phe Ala Ala Ala Leu Glu His His His His His His
20 25

<210> 10
<211> 90
<212> DNA
<213> Escherichia coli

<400> 10
atggttatgc ctggtgataa tattaaaatg gttgttactt taattcatcc tattgctatg 60

gatgatggtt tacgttttgc tattcgtgaa 90

<210> 11
<211> 135
<212> DNA
<213> Escherichia coli

<400> 11
gttggtccta ataatgttcc ttatattgtt gctactatta cttctaattc tgctggtggt 60

caacctgttt ctttagctaa tttaaaagct atgtattcta ttgctaaaaa atatgatatt 120

cctgttgtta tggat 135

<210> 12
<211> 93
<212> DNA
<213> Escherichia coli

<400> 12
aataattctt ggactcgtgt tgcttttgct ggtttaaaat ttcaagatgt tggttctttt 60

gattatggtc gtaattatgg tgttgtttat gat 93

<210>  13
<211>  90
<212>  DNA
<213>  Escherichia coli

<400>  13
ttaggttggt ctcaatatca tgatactggt tttattaata ataatggtcc tactcatgaa    60

aatcaattag gtgctggtgc ttttggtggt    90

<210>  14
<211>  60
<212>  DNA
<213>  Escherichia coli

<400>  14
gttggtccta ataatgttcc ttatattgtt gctactatta cttctaattc tgctggtggt    60

<210>  15
<211>  51
<212>  DNA
<213>  Escherichia coli

<400>  15
ttagctgttg gtttatatga tggtatgaat ttagattggt tagcttatcg t    51

<210>  16
<211>  96
<212>  DNA
<213>  Escherichia coli

<400>  16
aattatggtg ttgtttatga tgttacttct tggactgatg ttttacctga atttggtggt    60

gatacttatg gttctgataa ttttatgcaa caacgt    96

<210>  17
<211>  51
<212>  DNA
<213>  Escherichia coli

<400>  17
tttggtggtg atacttatgg ttctgataat tttatgcaac aacgtggtaa t    51

<210>  18
<211>  87
<212>  DNA
<213>  Escherichia coli

<400>  18
gatgctggta ttaatactga taatattgtt gctttaggtt tagtttatca atttgctgct    60

gctttagaac atcatcatca tcatcat    87

## Description

Technical Field

The present invention relates to peptides having an affinity for polydimethylsiloxane, and the use of the peptides.

Background Art

A variety of fields, including clinical examination, drug discovery research, environmental monitoring, and biochemistry, have widely used techniques to immobilize a protein, a nucleic acid, cells, etc., on a substrate for use in the detection, quantification, or analysis of a desired substance. For instance, a technique is known that immobilizes a protein such as an enzyme or an antibody on a substrate to detect a desired substance based on the enzymatic reaction or antigen-antibody reaction with the immobilized protein.

These techniques are still an active area of research today, and aim to enable highly accurate and highly efficient detection, quantification, analysis, etc. For example, a study reports a technique to treat the surface of a substrate by chemical processing or plasma processing, depending on the intended use. A peptide capable of immobilizing a protein on a polystyrene substrate (PTL 1) or a peptide capable of specifically and firmly immobilizing a protein on a polycarbonate substrate or polymethylmethacrylate substrate (PTL 2) are also reported.

Polydimethylsiloxane, one type of silicone rubber, can be microfabricated, and is thus known as one of the substrates for microchips, including microfluidic flow paths. Desirably immobilizing a protein on polydimethylsiloxane would enable highly accurate and highly efficient detection of a desired substance, etc., showing promise for further development of techniques using a polydimethylsiloxane substrate. However, peptides having an affinity for polydimethylsiloxane substrates are still unknown.

Citation List

Patent Literature

PTL 1: WO2009/101807A1
PTL 2: JP2011-168505A

Summary of Invention

Technical Problem

In view of the status quo of the art, an object of the present invention is to provide a polydimethylsiloxane (PDMS) substrate to which a peptide having an affinity for PDMS is bound. The present invention also provides a method for immobilizing a target protein on a PDMS substrate. Another object of the present invention is to provide a peptide having an affinity for PDMS. Another object of the present invention is to provide a polynucleotide that encodes a peptide having an affinity for PDMS. Another object of the present invention is to provide a vector using this polynucleotide, etc.

Solution to Problem

The present inventors conducted extensive research to solve the above problem, and found a peptide having an affinity for PDMS. The present invention has been completed on the basis of this finding. Specifically, the present invention includes the following.

Item 1. A polydimethylsiloxane substrate to which a peptide having an affinity for polydimethylsiloxane is bound, the peptide comprising the following peptide (1a) or (1b), or a fragment thereof:

(1a) a peptide comprising an amino acid sequence represented by at least one member selected from the group consisting of SEQ ID NOs: 1 to 9; and
(1b) a peptide comprising the amino acid sequence of (1a) in which one or more amino acids are deleted, substituted, and/or added, the peptide having an affinity for polydimethylsiloxane.

Item 2. The polydimethylsiloxane substrate according to Item 1, wherein the fragment comprises 15 to 58 amino acid residues.

Item 3. The polydimethylsiloxane substrate according to Item 1 or 2, on which a target protein is immobilized via the peptide having an affinity for polydimethylsiloxane.

Item 4. A method for immobilizing a target protein on a polydimethylsiloxane substrate, the method comprising the step of bringing a peptide having an affinity for polydimethylsiloxane into contact with the polydimethylsiloxane substrate,
wherein the peptide is incorporated in the target protein, and the peptide comprises the following peptide (1a) or (1b), or a fragment thereof:

(1a) a peptide comprising an amino acid sequence represented by at least one member selected from the group consisting of SEQ ID NOs: 1 to 9; and
(1b) a peptide comprising the amino acid sequence of (1a) in which one or more amino acids are deleted, substituted, and/or added, the peptide having an affinity for polydimethylsiloxane.

Item 5. A method for immobilizing a target protein on a polydimethylsiloxane substrate, the method comprising the step of binding the peptide having an affinity for polydimethylsiloxane bound to the polydimethylsiloxane substrate according to Item 1 or 2 to the target protein.

Item 6. A composition for immobilizing a target protein on a polydimethylsiloxane substrate, the composition comprising a peptide having an affinity for polydimethylsiloxane, the peptide comprising the following peptide (1a) or (1b), or a fragment thereof:

(1a) a peptide comprising an amino acid sequence represented by at least one member selected from the group consisting of SEQ ID NOs: 1 to 9; and
(1b) a peptide comprising the amino acid sequence of (1a) in which one or more amino acids are deleted, substituted, and/or added, the peptide having an affinity for polydimethylsiloxane.

Item 7. Use of a peptide having an affinity for polydimethylsiloxane in immobilizing a target protein on a polydimethylsiloxane substrate, the peptide comprising the following peptide (1a) or (1b), or a fragment thereof:

(1a) a peptide comprising an amino acid sequence represented by at least one member selected from the group consisting of SEQ ID NOs: 1 to 9; and
(1b) a peptide comprising the amino acid sequence of (1a) in which one or more amino acids are deleted, substituted, and/or added, the peptide having an affinity for polydimethylsiloxane.

Item 8. A peptide having an affinity for polydimethylsiloxane, the peptide comprising the following peptide (1c) or (1d), or a fragment thereof:

(1c) a peptide comprising an amino acid sequence represented by at least one member selected from the group consisting of SEQ ID NOs: 2 to 9; and
(1d) a peptide comprising an amino acid sequence represented by at least one member selected from the group consisting of SEQ ID NOs: 1 to 9 in which one or more amino acids are deleted, substituted, and/or added, the peptide having an affinity for polydimethylsiloxane.

Item 9. The peptide having an affinity for polydimethylsiloxane according to Item 8, wherein the fragment comprises 15 to 58 amino acid residues.

Item 10. A polynucleotide encoding the peptide having an affinity for polydimethylsiloxane according to Item 8 or 9.

Item 11. The polynucleotide according to Item 10, which is represented by at least one member selected from the group consisting of SEQ ID NOs: 10 to 18.

Item 12. A vector for expressing a peptide having an affinity for polydimethylsiloxane, the vector comprising the polynucleotide according to Item 10 or 11.

Item 13. A vector for expressing a peptide fusion protein,

the peptide fusion protein comprising the peptide having an affinity for polydimethylsiloxane according to Item 8 or 9 and a target protein,
the vector comprising the polynucleotide according to Item 10 or 11 and a polynucleotide encoding the target protein linked thereto.

Item 14. A transformant obtainable by introducing the vector according to Item 13 into a host cell to transform the host cell.

Item 15. A peptide fusion protein comprising the peptide having an affinity for polydimethylsiloxane according to Item 8 or 9 and a target protein, the peptide fusion protein being obtainable from the transformant according to Item 14.

Item 16. A linker for immobilizing a target protein on a polydimethylsiloxane substrate, the linker comprising the peptide having an affinity for polydimethylsiloxane according to Item 8 or 9.

Advantageous Effects of Invention

The peptide of the present invention has an affinity for PDMS. Because of the affinity, the peptide of the present invention makes it easy to immobilize a target protein on a PDMS substrate via the peptide. The present invention therefore enables a target protein to be highly accurately and highly efficiently immobilized on a PDMS substrate via the peptide having an affinity for PDMS.

Brief Description of Drawings

Fig. 1 illustrates the adsorption density of peptide fusion proteins on a PDMS substrate.
Fig. 2 illustrates the remaining activity of peptide fusion proteins on a PDMS substrate.
Fig. 3 illustrates a program of HPLC used in Example 3.
Fig. 4 illustrates the adsorption density of peptide fusion proteins on a PDMS substrate.
Fig. 5 illustrates the antigen-binding activity of peptide fusion proteins.
Fig. 6 illustrates the antigen-binding activity of peptide fusion proteins.

Description of Embodiments

The present invention provides a PDMS substrate to which a peptide having an affinity for PDMS is bound, and the like. The following describes the present invention.

1. Peptide Having Affinity for Polydimethylsiloxane (PDMS)

The peptide having an affinity for PDMS of the present invention comprises the following peptide (1-1) or (1-2), or a fragment thereof:

(1-1) a peptide comprising an amino acid sequence represented by at least one member selected from the group consisting of SEQ ID NOs: 1 to 9; and
(1-2) a peptide comprising the amino acid sequence of (1-1) in which one or more amino acids are deleted, substituted, and/or added, the peptide having an affinity for polydimethylsiloxane.

The "peptide" in the present invention refers to a peptide containing two or more amino acid residues bound through a peptide bond, including those called oligo peptides, polypeptides, and proteins, depending on the number of amino acid residues.

The peptide (1-1) is not limited as long as the peptide comprises an amino acid sequence represented by at least one member selected from the group consisting of SEQ ID NOs: 1 to 9. For example, the peptide may comprise an amino acid sequence represented by any of SEQ ID NOs: 1 to 9, or may comprise two or more amino acid sequences represented by any of SEQ ID NOs: 1 to 9, while having an affinity for PDMS.

In the peptide (1-2), the range of "one or more" is not particularly limited as long as the peptide has an affinity for PDMS. The range of "one or more" is, for example, 1 to 15, preferably 1 to 10, more preferably 1 to 5, still more preferably 1 to 4, particularly preferably 1 to 3, and yet more particularly preferably 1 or 2. Techniques to delete, substitute, and/or add one or more amino acids in a specific amino acid sequence are known.

An example of the peptide having such a deletion, substitution, and/or addition is a peptide comprising an amino acid sequence having at least 50% identity to the amino acid sequence represented by at least one member selected from the group consisting of SEQ ID NOs: 1 to 9 and having an affinity for PDMS. Another example is a peptide comprising an amino acid sequence having at least 50% identity to the amino acid sequence represented by any of SEQ ID NOs: 1 to 9, and having an affinity for PDMS. Of these peptides, more preferable peptides are those having at least 70% identity, preferably at least 80% identity, more preferably at least 90% identity, still more preferably at least 95% identity, particularly preferably at least 97% identity, and yet particularly preferably at least 98% identity to the amino acid sequences described above.

The fragment of peptide (1-1) or (1-2) is also not particularly limited as long as the fragment is a fragment of peptide (1-1) or (1-2) described above, and has an affinity for PDMS. Examples of the fragment include those containing 15 to 58 amino acid residues, preferably 15 to 45 amino acid residues, and more preferably 15 to 30 amino acid residues, and having an affinity for PDMS.

Without limiting the present invention, in an embodiment, a peptide comprising the amino acid sequence represented by SEQ ID NO: 5 can also be referred to as a fragment comprising the amino acid sequence represented by SEQ ID NO: 2. Without limiting the present invention, the peptide comprising the amino acid sequence represented by SEQ ID NO: 8 is identical to the peptide in which 15 out of 17 amino acid residues form an amino acid sequence represented by SEQ ID NO: 7.

Without the need to mention, in this specification, "a peptide comprising an amino acid sequence represented by at least one member selected from the group consisting of SEQ ID NOs: 2 to 9" and "a peptide comprising an amino acid sequence represented by at least one member selected from the group consisting of SEQ ID NOs: 1 to 9 in which one or more amino acids are deleted, substituted, and/or added, the peptide having an affinity for polydimethylsiloxane" can also be described substantially in the same manner as above, and also substantially the same as in the description below. Specifically, for example, "a peptide comprising an amino acid sequence represented by at least one member selected from the group consisting of SEQ ID NOs: 2 to 9" is not limited as long as the peptide comprises an amino acid sequence represented by at least one member selected from the group consisting of SEQ ID NOs: 2 to 9, and may be, for example, a peptide comprising an amino acid sequence represented by any of SEQ ID NOs: 2 to 9, or a peptide comprising two or more amino acid sequences represented by any of SEQ ID NOs: 2 to 9, while having an affinity for PDMS. Other features can also be described in the same manner.

The term "having an affinity for PDMS" is not particularly limited as long as the peptide can be directly bound to a PDMS substrate whose surface is unmodified. The binding conditions may suitably be determined in accordance with the type of peptide for use, or the properties of a target protein to be immobilized on the PDMS substrate via the peptide or the properties of a desired substance interactive with the target protein. For example, the binding (incubating) conditions described in the Examples below may be applied, or a person skilled in the art may suitably determine the conditions with reference to the conditions described in the Examples. The peptide can be bound to a PDMS substrate, for example, by bringing a solution, such as a buffer solution (e.g., a PBS solution) containing the peptide, into contact with the PDMS substrate for a predetermined period of time. The PBS used in the Examples described below was obtained by diluting 10×PBS (NaCl 1.38M (80.8 g), KCl 27 mM (2 g), $Na_2HPO_4 \cdot 12H_2O$ 80 mM (29 g), $KH_2PO_4$ 15 mM (2 g)) with ion-exchanged water to a volume of 1 L (a pH of 7.4). For example, when the PBS is suitably diluted, or its pH value is adjusted, the peptide can also be bound to a PDMS substrate.

The "PDMS substrate" is not limited as long as the substrate contains PDMS, with the surface of PDMS unmodified, on a part of the surface or on the entire surface of the substrate, and as long as the peptide having an affinity for PDMS can be bound to the surface of PDMS. Examples of PDMS substrates include substrates formed of PDMS and substrates formed by laminating and/or coating a part of the surface or the entire surface of a substrate formed of a different component/different components with PDMS.

The peptide having an affinity for PDMS of the present invention can be prepared by a known genetic engineering technique or chemosynthesis technique. For example, a desired peptide may be prepared by inserting a polynucleotide encoding a peptide represented by any of SEQ ID NOs: 1 to 9 into a vector, and then culturing the transformant into which the vector has been incorporated. The peptide having an affinity for PDMS of the present invention may also be obtained by isolating and/or purifying a peptide from a microorganism capable of producing the peptide. The peptide having an affinity for PDMS of the present invention may also be synthesized by a known chemosynthesis technique based on the information of the amino acid sequence represented by any of SEQ ID NOs: 1 to 9 or the nucleotide sequence encoding the amino acid sequence. The chemosynthesis technique includes peptide synthesis methods, such as liquid-phase peptide synthesis and solid-phase peptide synthesis. A more detailed example is the procedure described in the Examples below. Whether the obtained peptide has an affinity for PDMS can be determined based on whether the obtained peptide can be directly bound to a PDMS substrate whose surface is unmodified in the same manner as described above. If the peptide directly binds to the substrate, the peptide is determined to have an affinity for PDMS. The binding conditions can suitably be determined in the same manner as described above.

Because of the affinity for PDMS, the peptide having an affinity for PDMS of the present invention makes it easy to immobilize a target protein on a PDMS substrate via the peptide. This makes the peptide having an affinity for PDMS of the present invention highly useful in immobilizing a target protein on a PDMS substrate. The present invention also enables a target protein to be highly densely immobilized on a PDMS substrate via the peptide having an affinity for PDMS. The present invention also enables a target protein to be immobilized on a PDMS substrate, with the activity of the protein maintained. The present invention also enables a target protein to be immobilized on a PDMS substrate, controlling the uniformity of the protein orientation. Thus, the present invention can make a target protein on a PDMS substrate highly dense, highly activated, and/or highly orientationally controlled. This present invention enables a target protein to be highly accurately and highly efficiently immobilized on a PDMS substrate via the peptide having an affinity for PDMS, and further enables a desired substance interactive with the target protein to be highly accurately and highly efficiently bound to a PDMS substrate via the peptide having an affinity for PDMS. Thus, the peptide having an affinity for PDMS of the present invention is useful as a linker for immobilizing a target protein on a PDMS substrate.

The above indicates that the present invention provides a linker for immobilizing a target protein on a PDMS substrate, wherein the linker comprises the peptide having an affinity for PDMS; a composition for immobilizing a target protein on a PDMS substrate wherein the composition comprises the peptide having an affinity for PDMS; the use of the peptide having an affinity for PDMS in immobilizing a target protein on a PDMS substrate; and a kit comprising the composition for immobilization and a PDMS substrate. The peptide having an affinity for PDMS, the PDMS substrate, the target protein, the immobilization (binding) conditions, etc., for the linker for immobilization, the composition for immobilization, the use, and the kit, and effects obtained therefrom are explained in the same manner as described above.

In the linker for immobilization, the composition for immobilization, and the kit, the target protein may be one kind of protein, or two or more kinds of proteins.

In the use, the target protein may be one kind of protein, or two or more kinds of proteins.

As described above, the linker for immobilization is capable of immobilizing a target protein on a PDMS substrate via the peptide having an affinity for PDMS.

The composition for immobilization may comprise at least the peptide having an affinity for PDMS. Without limiting the present invention, in order to bind the peptide having an affinity for PDMS to a PDMS substrate in a simple operation, the composition may comprise a solvent, such as pure water, including ion-exchanged water, distilled water, ultrapure water, and RO water, and a buffer solution, including PBS; a target protein; and components necessary for immobilizing the target protein on a PDMS substrate via the peptide having an affinity for PDMS. An example of the solvent is preferably a buffer solution, and more preferably PBS. The peptide having an affinity for PDMS contained in the composition for immobilization is as described above. Without limiting the present invention, for example, when the peptide is produced by culturing a transformant containing a desired expression vector (e.g., *E. coli*), a peptide obtained after culture as is may be used as the peptide, and preferably a peptide obtained through a known purification treatment, such as dialysis, may also be used. The use of such a composition makes it simple to bind the peptide having an affinity for PDMS to a PDMS substrate, thus making it simple to immobilize a target protein on the PDMS substrate via the peptide having an affinity for PDMS.

These can suitably be used in the preparation of, for example, biochips, including protein chips, column packing materials, microplates for use in ELISA, and immobilized enzymes.

In this invention, the term "comprising" also encompasses the meanings "consisting essentially of" and "consisting of."

2. Polynucleotide

The present invention further provides a polynucleotide encoding the peptide having an affinity for PDMS. The polynucleotide of the present invention is not limited as long as it encodes the peptide having an affinity for PDMS described above, and examples of the polynucleotide include the following:

(2-1) a polynucleotide encoding the peptide having an affinity for PDMS,
(2-2) a polynucleotide comprising a nucleotide sequence represented by any of SEQ ID NOs: 10 to 18, and
(2-3) a polynucleotide hybridizing to a complementary strand of either polynucleotide (2-1) or polynucleotide (2-2) under stringent conditions and encoding a peptide having an affinity for PDMS.

A person skilled in the art can readily analyze and obtain polynucleotide (2-1) described above by a known method based on the amino acid sequence of the peptide having an affinity for PDMS.

The amino acid sequence encoded by polynucleotide (2-2) corresponds to any of amino acid sequences represented by SEQ ID NOs: 1 to 9.

In (2-3) described above, the expression "hybridizing ... under stringent conditions" indicates that two polynucleotide fragments can hybridize to each other under standard hybridization conditions. The conditions are disclosed in Sambrook et al., Molecular Cloning: A laboratory manual (1989), Cold Spring Harbor Laboratory Press, New York, USA.

More specifically, "stringent conditions" refers to hybridization in 6.0×SSC at about 45°C, followed by washing with 2.0×SSC at 50°C.

A polynucleotide that hybridizes to a complementary strand under stringent conditions typically has a certain degree or more of identity to either nucleotide sequence (2-1) or nucleotide sequence (2-2) described above. The polynucleotide has, for example, at least 70%, preferably at least 85%, more preferably at least 90%, still more preferably at least 95%, particularly preferably at least 98%, and yet more particularly preferably at least 99% identity to nucleotide sequence (2-1) or nucleotide sequence (2-2). The identity of the nucleotide sequence can be determined using a commercially available analytical tool or an analytical tool available through telecommunication (e.g., Internet). For example, software, such as FASTA, BLAST, PSI-BLAST, or SSEARCH, can be used for calculation.

The term "having an affinity for PDMS" is as described above, and is not limited as long as the peptide prepared using the polynucleotide described above can be directly bound to a PDMS substrate whose surface is unmodified. The binding conditions can also suitably be determined as described above. The preparation of a peptide from the polynucleotide described above may be performed using a genetic engineering technique or chemosynthesis technique known in the art, and this is easy for a person skilled in the art. For example, a peptide can be prepared by using, for example, an expression vector described below.

The polynucleotide of the present invention can also be prepared by a known genetic engineering technique or chemosynthesis technique (see Proc. Natl. Acad. Sci., USA., 78, 6613 (1981); Science, 222, 778 (1983); Molecular Cloning 2d Ed, Cold Spring Harbor Lab. Press (1989); Lectures on Biochemical Experiments (Genetic Research Methods I, II, III), Journal of The Japanese Biochemistry Society (1986)). For example, the polynucleotide may be obtained by preparing a cDNA library from a suitable source, such as microorganisms containing the desired polynucleotide, using a standard method, and obtaining the desired polynucleotide using a suitable probe from the library. Alternatively, a desired polynucleotide may be prepared and obtained by a known chemical DNA synthetic technique based on the sequence information of the amino acid represented by any of SEQ ID NOs: 1 to 9, or the sequence information of the nucleotide represented by any of SEQ ID NOs: 10 to 18.

## 3. Expression Vector

The present invention provides a vector for expressing a peptide having an affinity for PDMS, the vector comprising the aforementioned polynucleotide. The vector for expressing a peptide having an affinity for PDMS of the present invention is not particularly limited as long as the vector comprises the polynucleotide described above, and can express the peptide having an affinity for PDMS or the linker for immobilization described above in the host cell based on the base sequence of the polynucleotide. The vector is suitably selected based on its relationship with the host cell as typically performed in the art.

More specifically, the vector for use in the present invention is not limited as long as the vector is an expression vector typically used in the genetic engineering field. Examples thereof include plasmid vectors, such as pBR, pUC, pCD, pET, pGEX, pCMV, pMSG, and pSVL derived from bacteria such as *E. coli* or yeast; and viral vectors derived from retroviruses, adenoviruses, vaccinia viruses, baculoviruses, bacteriophages, etc.

These vectors optionally have a promoter linked thereto. The promoter is not limited as long as it is suitable for the host cell, and known promoters can be used. Examples of promoters include lac promoter, trp promoter, tac promoter, trc promoter, racA promoter, λPL promoter, lpp promoter, and T7 promoter; and these promoters are used, for example, when *E. coli* is used as the host cell. Other examples of promoters include SV40 promoter, CMV promoter, RSV promoter, HSV-TK promoter, LTR promoter, SRα promoter, and EF-1α promoter. These promoters are used, for example, when an animal cell is used as the host cell. With consideration for the relationship with the host cell, yeast cell promoters, insect cell promoters, and viral promoters may also be used. When the vector includes an endogenous promoter, the endogenous promoter may be used.

The promoter-binding site in the vector for expressing a peptide having an affinity for PDMS of the present invention is not limited, as long as the peptide having an affinity for PDMS can be expressed in the host cell. Typically, the promoter is linked to a site upstream of the polynucleotide encoding the peptide having an affinity for PDMS. More specifically, the polynucleotide encoding the peptide having an affinity for PDMS is under the control of the promoter in the vector for expressing the peptide having an affinity for PDMS of the present invention.

As a host cell, various known prokaryotic cells and eukaryotic cells may be used. Examples thereof include bacteria, such as *E. coli, Bacillus subtilis, Streptococcus, Staphylococcus*, actinomycetes, and filamentous fungi; yeast; *Aspergillus;* insect cells, such as *Drosophila S2,* and *Spodoptera Sf9;* and animal or plant cells, such as L cells, CHO cells, COS cells, Art-20 cells, HeLa cells, C127 cells, myeloma cells, GH3 cells, FL cells, VERO cells, CV-1 cells, Bowes melanoma cells, and oocytes of platanna.

These vectors, promoters, and host cells may suitably be combined based on the common technical knowledge in the art. Examples of combinations include pET (T7 promoter)/*E. coli* BL21 (DE3), and pGEX (Tac promoter)/*E. coli* BL21.

In addition, base sequences of, for example, marker genes, such as an enhancer, a splicing signal, a poly-A signal, a drug-resistant gene, green fluorescent protein (GFP), and others may further be bound to the vector for expressing a peptide having an affinity for PDMS of the present invention. These base sequences are bound at any site of the expression vector, depending on the intended use.

A base sequence constituting a linker for introducing a peptide having an affinity for PDMS into a target protein described later may further be bound to the vector for expressing a peptide having an affinity for PDMS of the present invention. For example, the base sequence constituting the linker may be bound to the 5' terminal and/or 3' terminal of the polynucleotide that encodes the peptide having an affinity for PDMS. The base sequence constituting the linker is not limited as long as the effects of the present invention can be achieved, and a person skilled in the art may suitably determine the base sequence within the general scope of investigation using a known technique. Examples of the linker include so-called flexible linkers, and an example of the amino acid sequences of flexible linkers is $(G_4S)_n$ (e.g., n = 1 to 4). When the linker is used, a nucleotide sequence capable of expressing the linker may suitably be bound to the vector.

In addition, a polynucleotide encoding the amino acid sequence of a target protein may further be linked to the polynucleotide encoding the peptide having an affinity for PDMS in the vector for expressing the peptide having an affinity for PDMS of the present invention. This enables a target protein incorporating the peptide having an affinity for PDMS to be expressed, and also enables a target protein incorporating the linker for immobilization to be expressed. An expression vector formed by binding a polynucleotide encoding the amino acid sequence of a target protein to the expression vector described above may be referred to as a vector for expressing a peptide fusion protein that comprises the peptide having an affinity for PDMS and the target protein.

In this specification, the target protein can be any protein, and without limiting the present invention, examples includes proteins, such as antigens, antibodies, enzymes, substrates, receptor proteins, and lectins. More specifically, examples thereof include, but are not limited to, glutathione transferases (GST: Glutathione S-Transferase), green fluorescent protein (GFP), alkaline phosphatases, peroxidases, luciferases, $\beta$-galactosidase, trypsin, chymotrypsin, thrombin, Factor Xa, angiotensin-converting enzymes, tyrosine kinases, insulin receptors, EGF receptors, maltose-binding proteins, monoclonal antibodies, polyclonal antibodies, single-chain antibodies, multivalent single-chain antibodies (e.g., bivalent single-chain antibodies), constant region fusion single-chain antibodies, Fab fragments and F(ab')2 fragments (fragments of antibodies including antigen-binding sites), complement protein C1q, concanavalin A, lentil lectin, antibody-binding proteins (e.g., protein A, ZZ, protein G, and protein L), biotin, and streptavidin (avidin).

When the nucleotide sequences encoding the amino acid sequences of these target proteins are known, a desired nucleotide sequence may be positioned in the expression vector by a known method based on the known sequence information. When the nucleotide sequence encoding the amino acid sequence of a target protein is unknown, a known genetic engineering technique or chemosynthesis technique may be used to analyze and prepare the polynucleotide encoding the amino acid sequence of the target protein based on the amino acid sequence of the target protein, and the prepared polynucleotide may be positioned in the expression vector.

To express the peptide fusion protein, the polynucleotide encoding the amino acid sequence of a target protein is also positioned under the control of the promoter. As long as this is done, a person skilled in the art may suitably determine whether to bind the polynucleotide encoding the target protein upstream or downstream of the polynucleotide encoding the peptide having an affinity for PDMS, or whether to bind the polynucleotide encoding the peptide having an affinity for PDMS to the interior of the molecule of the target protein. In any case, the polynucleotide is preferably linked to a site that does not adversely affect the physiological activity and conformation of the target protein. For example, when the target protein is an antigen, the polynucleotide is preferably positioned on a site that does not adversely affect antigen determination; when the target protein is an antibody, the polynucleotide may be positioned on a site that does not adversely affect antigen-binding; and when the target protein is an enzyme, the polynucleotide may be positioned on a site that does not adversely affect enzyme activity. A person skilled in the art may suitably determine the introduction site depending on the properties and structure of the target proteins, such as antigens, antibodies, enzymes, substrates, receptor proteins, and lectins. The polynucleotide encoding the peptide having an affinity for PDMS or the polynucleotide encoding the linker for immobilization is linked to a site that does not affect the properties of the peptide having an affinity for PDMS (e.g., the affinity for the substrate), and that does not adversely affect the effects of the present invention.

In the vector for expressing a peptide fusion protein of the present invention, the polynucleotide encoding the peptide having an affinity for PDMS is linked to the polynucleotide encoding the amino acid sequence of a target protein, and the linkage structure is not limited as long as a desired peptide fusion protein is expressed in the host cell. For example, in the vector for expressing a peptide fusion protein of the present invention, the polynucleotide encoding the peptide having an affinity for PDMS and the polynucleotide encoding the amino acid sequence of a target protein may be present as a continuous base sequence; more specifically, these polynucleotides may be directly linked without a linker. Alternatively, these polynucleotides may be linked to each other via any base sequence, for example, a base sequence constituting a linker, such as the aforementioned flexible linker. As long as the effects of the present invention can be achieved, the linkage structure is not limited. The polynucleotide encoding the amino acid sequence of a target protein in the present invention may be used singly or in a combination of two or more, as long as the effects of the present invention are not adversely affected.

The expression vector of the present invention may be prepared by a known method in the art, by positioning necessary base sequences, such as polynucleotides, on a suitable site of the vector using a restriction enzyme or the like.

4. Transformant

The present invention provides a transformant obtained by introducing the vector for expressing a peptide fusion protein to a host cell and transforming the cell.
In the present invention, examples of the host cell include those described above.
The method for obtaining a transformant by introducing the vector for expressing a peptide fusion protein into a host cell is not particularly limited, and a typical known method may be used. For example, the transformant may be obtained by methods described in various standard laboratory manuals. Specific examples thereof include a calcium chloride method; a rubidium chloride method; DEAE-dextran-mediated transfection; microinjection; cationic lipid-mediated transfection using, for example, a liposome; electroporation; transduction; and infection by bacteriophage.

5. Peptide Fusion Protein

The present invention provides a peptide fusion protein comprising the peptide having an affinity for PDMS and the target protein. The peptide having an affinity for PDMS and the target protein are as described above. The peptide fusion protein is a fusion protein unified by linking the peptide having an affinity for PDMS to the target protein.
Without limiting the present invention, in an embodiment, the peptide fusion protein can be prepared by culturing the transformant in a suitable medium, and collecting the desired peptide fusion protein from the transformant and/or culture. The culturing and collection methods are not particularly limited, and typical known methods may be used. For example, culturing may be performed by passage culture or batch culture using any medium commonly used for the host cell. The culturing may be continued until an adequate amount of peptide fusion protein can be obtained, with the amount of the protein produced inside and outside of the transformant as an index. The culture conditions, such as temperature and time, may be known conditions suitable for the host cell.
The peptide fusion protein thus obtained may further be optionally isolated or purified, by various isolation operations using its physical properties, chemical properties, or the like. Examples of isolation operations include solvent extraction, distillation, and various types of chromatography (see Biochemistry Data Book II, pp. 1175-1259, First Edition, First Printing, 1980, Tokyo Kagaku Dojin Co., Inc.; Biochemistry, 25(25), 8274 (1986); Eur. J. Biochem., 163, 313 (1987)). Since the peptide fusion protein of the present invention has an affinity for PDMS, the peptide fusion protein may be isolated and purified by contacting the product obtained from a culture medium or transformant with a PDMS substrate to bind the peptide having an affinity for PDMS to the PDMS substrate. When the peptide fusion protein is expressed using a transformant, the peptide fusion protein may sometimes be present as an inclusion body. In this case, the peptide fusion protein may be isolated or purified by suitably solubilizing the inclusion body, and contacting the solubilized protein with a PDMS substrate to bind the peptide having an affinity for PDMS to the PDMS substrate. When the conformation of the peptide fusion protein of the present invention has been altered, the peptide fusion protein may also be bound to the PDMS substrate with the conformation being altered. Conformational refolding may also optionally be performed with the peptide fusion protein being bound to the PDMS substrate to isolate or purify the protein.

6. PDMS substrate to which a peptide having an affinity for PDMS has been bound

The present invention provides a PDMS substrate to which a peptide having an affinity for PDMS has been bound. It comprises the peptide having an affinity for PDMS bound to the PDMS substrate. The peptide having an affinity for PDMS and PDMS substrate are as described above.
The shape of the PDMS substrate is also not limited as long as the peptide having an affinity for PDMS can be bound thereto, and the PDMS substrate may be in any shape, such as a plate shape, film shape (sheet), spherical, granular (bead shape), fibrous, microplate, or cylindrical. When the PDMS substrate of the present invention is used as a biochip, such as a protein chip, the PDMS substrate is preferably, for example, in the shape of a plate or a film (sheet).
The PDMS substrate to which the peptide having an affinity for PDMS has been bound can be prepared by contacting the peptide having an affinity for PDMS or the linker for immobilization to a PDMS substrate to bind the peptide having an affinity for PDMS to the PDMS substrate. The contacting conditions may suitably be determined depending on the type of peptide having an affinity for PDMS, or the properties of the target protein to be immobilized to the PDMS substrate via the peptide having an affinity for PDMS or the properties of a desired substance interactive with the target protein. For example, a solution obtained by mixing the peptide having an affinity for PDMS with an solvent, or a composition comprising the peptide having an affinity for PDMS for immobilizing a target protein to the PDMS substrate may be added dropwise to the PDMS substrate, or the PDMS substrate may be immersed in such a solution, and then the PDMS substrate may be allowed to stand for a predetermined time period. Unnecessary components not bound to the PDMS substrate can be removed by washing them off with a solvent such as a buffer solution or water. The binding conditions of the Examples described below may be applied, or a person skilled in the art may suitably determine the contacting conditions with reference to the binding conditions described in the Examples.

Because of the affinity for PDMS, the peptide having an affinity for PDMS can be directly bound to a PDMS substrate in the present invention.

A target protein may further be immobilized to the PDMS substrate to which the peptide having an affinity for PDMS has been bound according to the present invention via the peptide having an affinity for PDMS. The target protein is as described above. The target protein may be one kind of protein, or two or more kinds of proteins.

The immobilization is not limited as long as the target protein is immobilized to a PDMS substrate via the peptide having an affinity for PDMS. The target protein may be immobilized to the PDMS substrate by introducing the peptide having an affinity for PDMS into the target protein and contacting the result with the PDMS substrate. Alternatively, the target protein may be immobilized to a PDMS substrate by introducing the peptide having an affinity for PDMS bound to the PDMS substrate into the target protein. This can prepare a PDMS substrate to which a target protein has been immobilized via the peptide having an affinity for PDMS. The conditions for contacting with the PDMS substrate are as described above.

Introduction of the peptide having an affinity for PDMS to a target protein is not limited, as long as the introduction does not adversely affect the effects of the present invention, and as long as the peptide having an affinity for PDMS can be introduced. The peptide having an affinity for PDMS may be introduced to a target protein directly or via a linker. A person skilled in the art may suitably determine the linker as described above.

The peptide having an affinity for PDMS can be introduced into any site of a target protein, as long as the introduction does not adversely affect the activity and orientation of the target protein and the properties of the peptide having an affinity for PDMS (e.g., the affinity for the substrate), and as long as the effects of the present invention are not impaired. For example, when the target protein is an antigen, the peptide having an affinity for PDMS may be positioned on a site that does not adversely affect the antigen determination; when the target protein is an antibody, the peptide having an affinity for PDMS may be positioned on a site that does not adversely affect the antigen-binding; and when the target protein is an enzyme, the peptide having an affinity for PDMS may be positioned on a site that does not adversely affect the enzyme activity. A person skilled in the art may suitably determine the site depending on the properties and structure of the target protein, such as antigens, antibodies, enzymes, substrates, receptor proteins, or lectins. In particular, because the site to introduce the peptide having an affinity for PDMS can suitably be determined in the present invention, it is possible to uniformly control the orientation of the target protein to immobilize the protein on PDMS, while maintaining the activity of the protein.

The method for introducing the peptide having an affinity for PDMS into a target protein is not particularly limited, and the peptide may suitably be introduced by a known genetic engineering technique or chemosynthesis technique. For example, the peptide may be introduced by using the expression vector as described above. For example, the peptide having an affinity for PDMS may also be introduced into the target protein using a crosslinking agent, such as glutaraldehyde and NHS/EDC (N-hydroxysuccinimide/1-ethyl-3-(3-dimethylaminopropyl)carbodiimide hydrochloride). Alternatively, for example, the peptide having an affinity for PDMS may be introduced into a target protein by a specific bond via biotin-streptavidin (avidin), which is a bond between a biotinylated peptide having an affinity for PDMS and a streptavidin (avidin)-labeled target protein. For example, the peptide having an affinity for PDMS may also be introduced into a target protein by a specific bond via streptavidin (avidin), using a biotinylated peptide having an affinity for PDMS and a biotinylated target protein. The peptide may be introduced by a known method as described here.

A preferable example of the peptide having an affinity for PDMS introduced into a target protein is the peptide fusion protein described above. By contacting the peptide fusion protein with a PDMS substrate as described above, the target protein can be immobilized on the PDMS substrate via the peptide having an affinity for PDMS. Alternatively, by introducing the peptide having an affinity for PDMS into the target protein using the crosslinking agent or specific bond, and contacting the result with the PDMS substrate as described above, the target protein can be immobilized on the PDMS substrate via the peptide having an affinity for PDMS. This indicates that the present invention further provides a method for producing a target protein capable of binding to a PDMS substrate, comprising the step of introducing the peptide having an affinity for PDMS into the target protein, and also provides a target protein to which the peptide having an affinity for PDMS has been introduced.

The target protein can also be immobilized on a PDMS substrate via the peptide having an affinity for PDMS by introducing the peptide having an affinity for PDMS bound to the PDMS substrate into the target protein using the aforementioned crosslinking agent, specific bond, or the like.

The PDMS substrate to which the peptide having an affinity for PDMS has been bound according to the present invention enables the target protein to be immobilized on the substrate in a highly dense manner, with the activity of the protein being sufficiently maintained, and/or the orientation being uniformly controlled. Because of this feature, the PDMS substrate to which the peptide having an affinity for PDMS has been bound according to the present invention enables highly accurate and highly efficient detection, measurement, and analysis of a target protein or a desired substance interactive with the target protein.

Thus, if in the shape of a plate, film (sheet), or the like, the PDMS substrate to which the peptide having an affinity for PDMS has been bound according to the present invention can be used as a biochip, in particular, a protein chip. The PDMS substrate can also suitably be used as a column packing material using an antigen-antibody reaction, enzyme reaction, or the like, a microplate for use in ELISA, an immobilized enzyme, etc. The substrate has applications in a variety of fields, including clinical examination, drug discovery research, environmental monitoring, and biochemistry.

## 7. Method for immobilizing a target protein on a PDMS substrate

The present invention provides a method for immobilizing a target protein on a PDMS substrate, the method comprising the step of contacting the peptide having an affinity for PDMS introduced into the target protein with the PDMS substrate.

The target protein, the peptide having an affinity for PDMS, the PDMS substrate, introducing the peptide having an affinity for PDMS into the target protein, and contacting the peptide having an affinity for PDMS introduced into the target protein with a PDMS substrate are as described above.

Because of the affinity for PDMS of the peptide, the immobilization method of the present invention can bind the peptide having an affinity for PDMS to a PDMS substrate by simply contacting the peptide having an affinity for PDMS introduced into the target protein with the PDMS substrate. Thus, the immobilization method of the present invention can easily immobilize the target protein on the PDMS substrate via the peptide having an affinity for PDMS.

The immobilization method of the present invention may be performed by further combining the step of introducing the peptide having an affinity for PDMS into the target protein. More specifically, the step of contacting the peptide having an affinity for PDMS introduced into the target protein with the PDMS substrate can be performed after conducting the step of introducing the peptide having an affinity for PDMS into the target protein. The introduction and the like are also as described above.

Additionally, the present invention also provides a method for immobilizing a target protein on a PDMS substrate, comprising the step of binding the peptide having an affinity for PDMS bound to the PDMS substrate to the target protein.

The target protein, the peptide having an affinity for PDMS, the PDMS substrate, and binding the peptide having an affinity for PDMS to the PDMS substrate are as described above. The binding of the peptide having an affinity for PDMS bound to the PDMS substrate to the target protein is also not limited as long as the binding does not adversely affect the effects of the present invention. A person skilled in the art may suitably bind the peptide having an affinity for PDMS bound to the PDMS substrate to the target protein in accordance with common technical knowledge. For example, the introduction method described above may be used.

The immobilization method of the present invention may be performed by further combining the step of contacting the peptide having an affinity for PDMS with the PDMS substrate. More specifically, the step of binding the peptide having an affinity for PDMS bound to the PDMS substrate to the target protein can be performed after performing the step of contacting the peptide having an affinity for PDMS with the PDMS substrate to bind them. Contacting the peptide having an affinity for PDMS with the PDMS substrate may be performed as described above.

These immobilization methods can produce a PDMS substrate on which a target protein has been immobilized in a simple manner. Because the target protein is immobilized on a PDMS substrate via the peptide having an affinity for PDMS, these immobilization methods can immobilize the target protein in a highly dense manner, with the activity of the protein being sufficiently maintained and/or the orientation of the protein being uniformly controlled. Thus, the present invention makes it easy to produce biochips such as protein chips, column packing materials using an antigen-antibody reaction, enzyme reaction, or the like, microplates for use in ELISA or other assays, and immobilized enzymes. The immobilization method of the present invention is thus useful in various fields, such as clinical examination, drug discovery research, environmental monitoring, and biochemistry.

## Examples

The following describes the present invention with reference to Examples; however, the present invention is not limited to these Examples.

### Example 1

The affinity of the peptide represented by SEQ ID NO: 1 (ELV1 peptide), the affinity of the peptide represented by SEQ ID NO: 2 (TPV1 peptide), and the affinity of the peptide represented by SEQ ID NO: 3 (OCV1 peptide) for polydimethylsiloxane (PDMS) were examined in accordance with the procedure described below.

1. Procedure

1-1. Construction of elongation factor Tu (ELN) expression vector, tryptophanase (TPA) expression vector, and outer membrane protein C (OMC) expression vector

1) Chromosomal DNA of *E. coli* BL21(DE3) (Novagen) was extracted using a DNA purification kit (Promega Corporation).
2) PCR was performed with the chromosomal DNA as a template using KOD plus ver. 2 PCR kit (Toyobo Co., Ltd.) to amplify the ELN gene.
3) The amplified ELN gene was mixed with a pET-22 vector (Novagen) to give a molar ratio of 3:1, and the same amount of Ligation High (Toyobo Co., Ltd.) as that of the mixture solution was added thereto, followed by inserting the amplified ELN gene between the *Nde* I site and the *Not* I site for cloning.
4) *E. coli* HST08 Premium (Takara Bio Inc.) was transformed with the above vector and cultured undisturbed overnight on an LB-Amp agar medium.
5) 2 mL of an Amp.-containing LB medium (Nacalai Tesque, Inc.) was taken with a 15-mL tube. A single colony from the agar plate was inoculated thereto, and cultured at 37°C and 200 rpm overnight.
6) After culture, the vector was collected and purified by alkaline SDS.
7) Incorporation of the gene was confirmed by agarose electrophoresis, and the nucleotide sequence of the introduced ELN gene was also confirmed by DNA sequence analysis (Greiner).
8) *E. coli* Rosetta (DE3) was transformed with the vector obtained in step 6) and cultured, thereby preparing an ELN expression *E.*

*coli.*

An OMC expression vector was prepared in the same manner as for the ELN expression vector, except that the ELN gene was replaced by OMC gene. A TPA expression vector was prepared in the same manner as for the ELN expression vector, except that the entire gene of TPA was synthesized by outsourcing (FASMAC), and that the gene amplified by PCR using a KODplus ver. 2 PCR kit (Toyobo Co., Ltd.) was used.

1-2. Construction of GST (glutathione-S-transferase) expression vector pGEX-3X

1) 1 μL of a GST expression vector (pGEX-3X) (GE Healthcare) was added to 50 μL of *E. coli* HST08 Premium Competent cells (Takara Bio Inc.), and the mixture was incubated on ice for 10 minutes.
2) The mixture was incubated at 42°C for 45 seconds and cooled on ice again.
3) The transformed *E. coli* was inoculated into an Amp.-containing LB agar plate, followed by culture at 37°C overnight.
4) 2 mL of an Amp.-containing LB medium was taken with a 15-mL tube, and a single colony from the agar plate was inoculated thereinto, followed by culture at 37°C and 200 rpm overnight.
5) Centrifugation was performed at 10,000 g for 10 minutes, and the supernatant was removed.
6) pGEX-3X was collected by alkaline SDS.
7) 10 μL of Cut Smart Buffer (Nacalai Tesque, Inc.), 2 μL of CIAP (Calf Intestine Alkaline Phosphatase, Toyobo Co., Ltd.), 2 μl of Eco RI-HF (New England Biolabs), and 2 μL of Bam HI-HF (New England Biolabs) were added to 20 μL of the collected vector solution, and the mixture was incubated at 37°C overnight, followed by cleavage and dephosphorylation of the vector. The cleavage state of pGEX-3X was confirmed by agarose electrophoresis.
8) The vector subjected to enzyme treatment was purified using illustra™GFXTM PCR DNA and Gel Band Purification Kit (GE Healthcare).

1-3. Preparation of GST fused with ELV1 peptide, TPV1 peptide, or OCV1 peptide

1) From the amino acid sequence of ELV1 peptide, the nucleotide sequence encoding this gene was determined, and oligo DNA was synthesized by outsourcing. The nucleotide sequence encoding ELV1 peptide is represented by SEQ ID NO: 10.
2) The nucleotide sequence obtained in 1) was cloned between the *Bam* HI site and the *Eco* RI site of the GST expression vector pGEX-3X constructed as described above, and the nucleotide sequence encoding ELV1 peptide introduced into the vector was confirmed by DNA sequence analysis.

3) *E. coli* BL21 (DE3) was transformed with the constructed expression vector and cultured overnight in 10 mL of a 2xYT medium (Novagen) containing ampicillin (Amp., Nacalai Tesque, Inc.).

4) The cultured medium was added to 50 mL of the same medium as in step 3) until $OD_{600}$ = 0.1 was achieved, and the mixture was cultured at 37°C and 200 rpm until $OD_{600}$ = 1.0 was achieved (about 1.5 hours).

5) 5 μL of 1M IPTG (Isopropyl-β-D(-)-thiogalactopyranoside, Wako Pure Chemical Industries, Ltd.) was added thereto, and the mixture was further cultured at 30°C and 200 rpm for 7 hours.

6) After culture, centrifugation was performed at 4500 rpm for 20 minutes, and the supernatant was removed.

7) 3 mL of BugBuster (BugBuster Protein Extraction Reagent, Novagen), 1.5 μL of Benzonase nuclease (Novagen), and 3 mg of lysozyme (Seikagaku Corporation) were added to the bacterial cells, and the mixture was stirred well and incubated at 37°C for 1 hour for lysis of the bacterial cells.

8) Centrifugation was performed at 10000 rpm for 20 minutes, and the supernatant was collected as a soluble fraction.

9) The soluble fraction was applied to a GSTrap HP column (GE Healthcare), and the interior of the column was washed with PBS containing 1 mM DTT (Dithiothreitol, Nacalai Tesque, Inc.).

10) ELV1 peptide fusion GST was collected by gradient elution with 100 mM Tris-HCl (pH 8.0) containing 20 mM reduced glutathione.

11) The eluate was dialyzed with PBS overnight, and the concentration was quantified by a DC Protein Assay Kit (Bio-Rad Laboratories, Inc.).

The PBS used in Example 1 was prepared before use by diluting a pre-prepared 10×PBS (NaCl (80.8 g) 1.38 M, KCl (2 g) 27 mM, $Na_2HPO_4 \cdot 12H_2O$ (29 g) 80 mM, $KH_2PO_4$ (2 g) 15 mM) to a volume of 1 L with ion-exchanged water.

TPV1 peptide fusion GST and OCV1 peptide fusion GST were also prepared from TPV1 peptide and OCV1 peptide, respectively. The nucleotide sequence encoding TPV1 peptide was represented by SEQ ID NO: 11, and the nucleotide sequence encoding OCV1 peptide was represented by SEQ ID NO: 12.

The steps after step 3) were performed in the same manner, except that the constructed expression vector was replaced by GST expression vector pGEX-3X constructed in section 1-2, thereby preparing a wild-type GST.

1-4. Adsorption of wild-type GST (wt-GST), ELV1 peptide fusion GST, TPV1 peptide fusion GST, and OCV1 peptide fusion GST on a PDMS substrate

1) PDMS (Sylgard® 184 Silicone Elastomer, Dow Corning) was dissolved in chloroform, and a 2.5 w/v% PDMS solution was prepared.

2) 60 μL of the PDMS solution prepared in step 1) was added onto a QCM (quartz crystal microbalance) sensor chip (QCMST27C, Initium, Inc.), and a PDMS thin film was formed by spin coating (6000 rpm, 1 minute). The film was then determined to serve as a PDMS substrate (approximate surface area: 81$cm^2$, area/volume ratio: 27 $cm^{-1}$).

3) The obtained PDMS substrate was set in the QCM device (Affinix QN, Initium, Inc.), and incubated in PBS until the baseline was stabilized.

4) The wt-GST solution or each of the peptide fusion GST solutions (hereinafter, "GST solutions") was added onto the substrate to sequentially give a final concentration of 0.1, 0.4, 1.3, 3.4, or 9.7 μg/ml, and each substrate was monitored for 1 hour for each. The GST solutions were prepared by adding the wt-GST or each peptide fusion GST to PBS.

5) From the measurement data, their adsorption density was calculated (-1Hz=0.62 ng/$cm^2$). The adsorption density (μg/$cm^2$) was calculated by applying the adsorption density and mol concentration to the following equation.

$$\frac{C_A}{q} = \frac{1}{q_{max}} \times C_A + \frac{K_d}{q_{max}}$$

$C_A$:GST Solution Final Mol Concentration(M) q:Adsorption Density (μg/$cm^2$)
$q_{max}$:Saturated Adsorption Density (μg/$cm^2$) $K_d$:Dissociation Constant (nM)

2. Results

Fig. 1 shows the results.

Fig. 1 shows the adsorption density on the PDMS substrate. As is clear from Fig. 1, ELV1 peptide fusion GST, TPV1 peptide fusion GST, and OCV1 peptide fusion GST exhibited remarkable improvement in adsorption density on the PDMS substrate as compared with wt-GST. This indicates that the introduction of the peptides represented by SEQ ID NOs: 1 to 3 made it possible to easily and densely immobilize GST on the PDMS substrate. Although not shown here, the use of OAT1 peptide fusion GST instead of ELV1 peptide fusion GST also exhibited remarkable improvement in adsorption density on the PDMS substrate as compared with wt-GST. OAT1 peptide is a peptide represented by SEQ ID NO: 4, and the nucleotide sequence encoding OAT1 peptide is represented by SEQ ID NO: 13. OAT1 peptide fusion GST was also prepared in the same manner as described above.

The results indicate that peptides represented by SEQ ID NOs: 1 to 4 have excellent affinity for a PDMS substrate, and are useful in immobilizing a target protein on a PDMS substrate. The peptides can also be introduced into a desired site of GST as described above, and thus-obtained peptide fusion GST exhibited improvement in adsorption density. This indicates that the peptides can maintain the activity of a target protein and control the orientation of the protein.

The examination of the dissociation constant Kd (nM) revealed that the dissociation constant of ELV1 peptide fusion GST from the PDMS substrate was 5.7 nM, while the dissociation constant of ELV1 peptide fusion GST from a substrate other than PDMS (e.g., a silicon nitride substrate) was 82 nM. A smaller dissociation constant indicates a lower likelihood of the peptide dissociating from a substrate. ELV1 peptide has an affinity for a PDMS substrate that is about 14 times higher than for a silicon nitride substrate, indicating that ELV1 peptide has a higher affinity for a PDMS substrate than for a silicon nitride substrate.

## Example 2

In accordance with the following procedure, a study was performed to examine whether a peptide fusion GST immobilized on a PDMS substrate maintains the activity inherent to GST.

### 1. Procedure

#### 1.1. Construction of Peptide Fusion GST

This Example used ELV1 peptide fusion GST, TPV1 peptide fusion GST, OCV1 peptide fusion GST, OAT1 peptide fusion GST, and wt-GST prepared in Example 1.

#### 1-2. Measurement of GST Remaining Activity

#### 1. Procedure

1) GST solutions of wt-GST, ELV1 peptide fusion GST, TPV1 peptide fusion GST, OCV1 peptide fusion GST, and OAT1 peptide fusion GST were diluted with PPB to prepare 2940 μL of each GST solution such that the GST concentration was 0.5 μg/ml when 30 μL of 100 mM CDNB and 30 μL of 100 mM GSH were added thereto.
2) 30 μL of 100 mM CDNB and 30 μL of 100 mM GSH were added thereto, and the absorbance at 340 nm was measured at 25°C for 3 minutes with a spectrophotometer (V-630BIO, JASCO Corporation), followed by calculation of a change in absorbance ($min^{-1}\cdot cm^{-1}$).
3) From the following equation, the value of specific activity of each GST before adsorption was calculated. From the molar absorbance coefficient $\varepsilon$=9.6 $mM^{-1}\cdot cm^{-1}$ of the product CDNB-GSH, the amount of the product generated in 1 minute was calculated, and the result was determined to be enzyme activity.

$$\text{Specific Activity}\,(\mu mol\cdot min^{-1}\cdot mg^{-1}) = \frac{\text{Change in Absorbance}\,(min^{-1}\cdot cm^{-1})\times 10^{3}}{\text{Molar Absorbance Coefficient}\,(mM^{-1}\cdot cm^{-1})}$$

4) GST solutions of wt-GST, ELV1 peptide fusion GST, TPV1 peptide fusion GST, OCV1 peptide fusion GST, and OAT1 peptide fusion GST were diluted with PBS, and adjusted to prepare 2 mL of each to give 100 μg/mL. These solutions were individually mixed with a PDMS substrate (approximate surface area: 54 $cm^2$, area/volume ratio: 27 $cm^{-1}$), and adsorption was performed at 25°C for 2 hours.
5) The supernatants were removed, and the PDMS substrates were washed with PBS three times and with PPB once.

6) 2940 μL of PPB, 30 μL of 100 mM CDNB, and 30 μL of 100mM GSH were added to the washed PDMS substrates. Changes in absorbance at 340 nm were measured every 30 minutes with stirring at 37°C and 300 rpm with NanoDrop (Thermo), and changes in absorbance were calculated ($min^{-1} \cdot cm^{-1}$).
7) The value of the specific activity of each GST after adsorption was calculated from the equation above, and the remaining activity was calculated from the values of the specific activity before and after adsorption.

$$\text{Remaining Activity (\%)} = \frac{\text{Specific Activity After Adsorption}}{\text{Specific Activity Before Adsorption}} \times 100$$

PPB for use was prepared by diluting 0.1M $KH_2PO_4$ to a volume of 1 L with ion-exchanged water and adjusting the pH to 6.5 with KOH.

2. Results

Fig. 2 shows the results. As is clear from Fig. 2, GST constituting peptide fusion GST maintained the activity inherent to GST even after immobilization on the PDMS substrate. This confirms that even when immobilized on a PDMS substrate, a target protein to which the peptide having an affinity for PDMS is bound exhibits its high activity.

Example 3

The affinity of each of the peptides represented by SEQ ID NOs: 1 to 9 for a PDMS substrate was examined in accordance with the following procedure.

1. Procedure

1) A PDMS substrate (approximate surface area: 81 $cm^2$, area/volume ratio: 27$cm^{-1}$), which was the same as the substrate in Example 1, was mixed with each of PBS solutions containing the peptides represented by SEQ ID NOs: 1 to 9, and each of the mixtures was shaken at 25°C and 200 rpm for 2 hours. The peptides represented by SEQ ID NOs: 1 to 4 are as described above. The peptide represented by SEQ ID NO: 5 was defined as TPV2 peptide, the peptide represented by SEQ ID NO: 6 was defined as TPT1 peptide, the peptide represented by SEQ ID NO: 7 was defined as OCT2 peptide, the peptide represented by SEQ ID NO: 8 was defined as OCV2 peptide, and the peptide represented by SEQ ID NO: 9 was defined as OCT3 peptide.
2) A portion of the supernatant of each solution was analyzed by HPLC, and the remaining solution of each was further mixed with the PDMS substrate. To perform this, the area/volume ratio per mL of the substrate was set to 27 $cm^{-1}$.
3) Step 2) was repeated three times in total.
4) Because a peptide that exhibits a significantly reduced peak area after adsorption can be determined to have an affinity for the PDMS substrate, chromatograms before and after adsorption were compared.

In step 2), the analysis by HPLC was performed as follows. First, an HPLC system was started up, and Lines A and B were replaced by HPLC liquids A and B. HPLC liquid A was then fed to a column at a flow rate of 1 ml/min to equilibrate the column. Subsequently, the PBS solution that had not been applied to the experiment yet and a portion of the supernatant collected in step 2) were individually filtered through a pretreatment filter, and 500 μL thereof was fed to the column. The concentration of liquid B was linearly increased in accordance with the program shown in Fig. 3 below to elute the peptide from the column. Thereafter, chromatograms before and after adsorption were compared.
The following is the details of the HPLC system, liquid A, liquid B, pretreatment filter, and program.

HPLC system

PU-2089 Quaternary Gradient Pump (JASCO International Co., Ltd.) LC-NetII/ADC (JASCO International Co., Ltd.)
MD-2018Plus Photodiode Array Detector (JASCO International Co., Ltd.)
UV-1575 Intelligent UV/VIS Detector (JASCO International Co., Ltd.)

TSKgel ODS-100Z 3 μm (column size: 4.6 mm, I.D. × 15 cm) (Tosoh Corporation)

Liquid A

Ultrapure Water (1 L)
TFA (Trifluoroacetic acid for high-performance liquid chromatography, Wako Pure Chemical Industries, Ltd.) (1 ml) 0.1v/v%

Liquid B

Acetonitrile (Chromasolv for HPLC, gradient grade, ≥ 99.9%) (Sigma-Aldrich Japan) (1 L)
TFA (for high-performance liquid chromatography) (1 ml) 0.1v/v%

Pretreatment Filter

Non-Sterile 4 mm Millex® HV syringe Driven Filter Unit (450 nm) (Millipore Corporation)

Program

Fig. 3 shows the program.

2. Results

Table 1 shows the results.

Table 1

| Peptide | Decrease (%) |
|---|---|
| ELV1 | 45 |
| TPV1 | 97 |
| OCV1 | 63 |
| OAT1 | 60 |
| TPV2 | 88 |
| TPT1 | 86 |
| OCT2 | 45 |
| OCV2 | 60 |
| OCT3 | 69 |

As is clear from Table 1, the use of any solution exhibited a decrease in peak area after adsorption, indicating that all of the peptides represented by SEQ ID NOs: 1 to 9 have an affinity for a PDMS substrate. This reveals that the peptides having these amino acid sequences make a target protein on a PDMS substrate via these peptides highly dense, highly activated, and highly orientationally controlled.

Example 4

In accordance with the following procedure, the peptide represented by SEQ ID NO: 1 (ELV1 peptide), the peptide represented by SEQ ID NO: 2 (TPV1 peptide), and the peptide represented by SEQ ID NO: 3 (OCV1 peptide) were individually linked to a single-chain antibody to C-reactive protein (CRP), and the obtained peptide fusion proteins were evaluated for their affinity for a PDMS substrate and activity.

1. Procedure

1-1. Preparation of Peptide Fusion scFv

1-1-1. Construction of Vector Incorporating ELV1 peptide, TPV1

Peptide, or OCV1 Peptide

1) A sense strand having a nucleotide sequence (SEQ ID NO: 10) encoding the amino acid sequence represented by SEQ ID NO: 1 and an antisense strand that has a complementary sequence to the sequence were synthesized by outsourcing.
2) The sense strand and the antisense strand were individually diluted with sterile water to give 10 pmol/μL, and 1 μL of each strand was mixed with 2 μL of a 10×High buffer (Toyobo Co., Ltd.) and 16 μL of sterile water. The mixture solution was incubated at 90°C for 5 minutes, and then the incubator was turned off, followed by cooling to room temperature, thereby preparing double-stranded DNA for ELV1 peptide.
2) pET-22 vector-bearing *E. coli* (pET-22-XL1 Blue, Novagen) was inoculated into 10 ml of an Amp.-containing LB medium, and cultured at 37°C at a rotation frequency of 200 rpm overnight.
The plasmid was collected by a miniprep, and digested with restriction enzymes NotI and XhoI.
3) The double-stranded DNA (insert) prepared in step 1) and pET-22 vector (which had been treated with a restriction enzyme beforehand) were mixed to give a molar ratio, insert:vector = 3:1, and incubated at 50°C for 10 minutes. The temperature was decreased to 16°C, and the same amount of Ligation high ver. 2 (Toyobo Co., Ltd.) as that of the mixture solution was added thereto, and incubated for 3 hours.
4) The sample after ligation was added to *E. coli* HST08 Premium Competent cells, and incubated at 42°C for 45 seconds to transform *E. coli.*
5) The transformed *E. coli* was inoculated into an Amp.-containing LB agar medium, and incubated at 37°C overnight. Six colonies expressed on the agar medium were picked up and inoculated into 2 ml of an Amp.-containing LB medium, and cultured at 37°C at a rotation frequency of 200 rpm overnight.
6) 2 ml of the obtained culture solution was placed in an Eppendorf tube, and centrifuged (4°C, 10000 rpm, 15 minutes) to remove the supernatant. Further, the plasmid DNA was collected by alkaline SDS. A restriction enzyme XhoI was added to a portion of the collected plasmid DNA, and whether it was cleaved was confirmed by agarose gel electrophoresis. The sequence of the uncleaved sample was confirmed by outsourcing the DNA analysis.

1-1-2. Construction of Peptide Fusion scFv Vector

1) PCR was performed using the single-chain antibody to CRP as a model scFv, and scFv gene was amplified.
2) The pET-22 vector bearing DNA that encodes ELV1 peptide obtained in section 1-1-1 above and scFv amplified by PCR were digested with restriction enzymes NdeI and NotI.
3) Subsequently, ligation, transformation, and culture were performed in the same manner as in steps 3) to 6) in section 1-1-1, and the plasmid was collected by a miniprep. The band was confirmed by agarose gel electrophoresis, and then the sequence was confirmed by outsourcing the DNA analysis.

1-1-3. Production, Purification, and Expression Confirmation of

Peptide Fusion scFv

1) The peptide fusion scFv vector obtained in section 1-1-2 was added to *E. coli* Rosetta (DE3), which was then transformed and inoculated into 10 ml of an Amp.-containing 2×YT medium, followed by preculture at 37°C and 200 rpm overnight.
2) The culture solution of step 1) was added to a 500 ml-Erlenmeyer flask equipped with a baffle containing 50 ml of an Overnight Express TB medium (OE medium, Nacalai Tesque) containing Amp. and chloramphenicol (Cm.)

such that $OD_{600}$=0.1 was achieved, and the mixture was cultured at 37°C and 200 rpm overnight. The culture solution was transferred to a 50 ml-centrifuge tube, and centrifuged at 10000 rpm for 20 minutes, thereby removing the supernatant.

3) 10 mL of 1×PBS, 100 μL of Triton X-100, and 100 μL of Protease inhibitor Cocktail (Nacalai Tesque) were added to the bacteria in the pellet form, and the mixture was vortex-mixed, followed by immersing the tip of an ultrasonic homogenizer into the solution to perform ultrasonic fragmentation for 4 minutes 3 times (output 50W, DUTY 30%).

3) Subsequently, centrifugation (4°C, 10000 rpm, 15 minutes) was performed, and the supernatant was removed. 5 mL of ion-exchanged water was added, and the mixture was vortex-stirred, followed by centrifugation again (4°C, 10000 rpm, 15 minutes). This operation was repeated twice, and the supernatant was removed. 3 mL of ion-exchanged water was added, and the mixture was suspended with vortex, followed by freezing at -80°C, and further freeze-drying overnight.

4) Subsequently, 5 mL of a solubilizing buffer containing 6M guanidine hydrochloride (6M guanidine hydrochloride, 2×PBS, pH 7.5) was added to the freeze-dried product, and the inclusion body was solubilized, followed by centrifugation (4°C, 10000 rpm, 15 minutes), thereby collecting the supernatant.

5) ELV1 peptide fusion scFv was purified by metal-chelate affinity chromatography under the following conditions.

Column: His-Trap HP

Binding buffer (per liter): urea 8M (480 g), imidazole 20 mM (1.36 g), 10×PBS 200 mL (adjusted with HCl to a pH of 7.5, and made up to a volume of 1 liter with deionized water)

Elution buffer (per liter): urea 8M (480 g), imidazole 400 mM (27.2 g), 10×PBS 200 mL (adjusted with HCl to a pH of 7.5, and made up to a volume of 1 liter with deionized water)

TPV1 peptide fusion scFv and OCV1 peptide fusion scFv were also prepared from TPV1 peptide and OCV1 peptide in the same manner. As a control, D10 peptide fusion scFv was prepared in the same manner, except that D10 peptide (a peptide having 10 contiguous aspartic acid residues) was used instead of ELV1 peptide. The individual peptide fusion scFv had a peptide sequence in 3' terminal of which 6 histidine residues were contiguously present.

1-2. Refolding and Recovery of Peptide Fusion scFv

1) Peptide fusion scFv was diluted with 8M urea-PBS such that the final concentration of scFv after purification was 1 mg/ml, that DTT was 100 mM, and that the total volume was 2 ml, and incubated at 4°C for 2 hours.

2) The solution in step 1) was placed in a dialysis membrane, and dialyzed at 4°C for 65 hours with 8M urea and 1 L of 50 mM Tris-HCl (pH 8.5) as an external solution, followed by DTT removal and air oxidation (the external solution was replaced in the middle).

3) The external solution was replaced by 1 L of 50 mM Tris-HCl (pH 8.5), and dialysis was performed, followed by removing the remaining urea (the external solution was replaced in the middle).

4) The internal solution after dialysis was centrifuged at 4°C and 20000 rpm for 5 minutes, and the supernatant was recovered. The concentration of the recovered peptide fusion scFv was calculated using a DC Protein Assay Kit.

5) The recovery rate was determined by dividing the concentration of the recovered peptide fusion scFv by the concentration of peptide fusion scFv before dialysis, and multiplying the result by 100.

1-3. Adsorption of Peptide Fusion scFv on a PDMS Substrate

1) In the same manner as in section 1-4 of Example 1, PDMS was dissolved in chloroform, and a 2.5 w/v% PDMS solution was prepared. This solution was applied onto a QCM sensor chip with spin coating (6000 rpm, 1 min) to form a PDMS thin film. This was determined to serve as a PDMS substrate.

2) The obtained PDMS substrate was set in the QCM device, and incubated in PBS until the baseline was stabilized. Each of the peptide fusion scFv solutions was added to give a final concentration of 0.5, 1.8, 4.9, 12.7, and 32.3 μg/ml, and the adsorption behavior at each concentration was monitored at 25°C at a rotation frequency of 1000 rpm for 1 hour. The solutions were prepared by adding each peptide fusion scFv to PBS.

3) The adsorption density was calculated from the measurement data obtained in step 2) ($\Delta F$:-1Hz=0.62 $ng/cm^2$).

1-4. Antigen-binding Activity of Peptide Fusion scFv

1) 1 μg/mL of CRP (antigen) (diluted with 1×PBS) was immobilized on a Maxisorp microplate (Thermo Fisher Scientific Inc.) (4°C, overnight).
2) The plate was washed with 0.1% PBST (PBS-0.1% Tween20), and 2% BSA-PBST was added thereto, followed by blocking at 25°C for 1 hour.
3) The plate was washed with 0.1% PBST, and peptide fusion scFv serially diluted to 0 to 100 μg/mL with 0.2% BSA-PBST was added thereto, followed by incubation at 25°C for 1 hour.
4) The plate was washed with 0.1% PBST, and a HRP (horseradish peroxidase)-labeled Anti 6×His antibody diluted with 0.2% BSA-PBST by a factor of 5000 was added thereto, followed by incubation at 25°C for 1 hour.
5) The plate was washed with 0.1% PBST, and a TMB substrate solution was added thereto to develop color (25°C, 15 minutes). 0.3M $H_2SO_4$ was added thereto, and the absorbance of the dominant wavelength at 450 nm and the complementary wavelength at 650 nm was measured.

1-5. Antigen-Binding Activity of Peptide Fusion scFv Immobilized

on a PDMS Substrate

1) A PDMS substrate was prepared in the same manner as above.
2) The PDMS substrate was set in the QCM device, and incubated in PBS until the baseline was stabilized. 15 μg/mL of each of the peptide fusion scFv solutions was added thereto, and immobilized at 25°C at a rotation frequency of 1000 rpm. The solutions were prepared by adding peptide fusion scFv to PBS.
3) Subsequently, PBS was replaced by 0.2% BSA-PBST, and CRP was added to give a final concentration of 0.1, 1.1, and 6.1 μg/mL. The adsorption behavior until equilibrium was monitored at each concentration in the same manner as above, and the amount of antigen-binding (ng/cm$^2$) was calculated from the measurement data (ΔF:-1Hz=0.62 ng/cm$^2$).

2. Results

2-1. Recovery Concentration and Recovery Efficiency of Peptide Fusion scFv

Table 2 shows the results of section 1-2, specifically the recovery concentration and recovery efficiency of peptide fusion scFv.

Table 2

| | D10 Peptide Fusion scFv | ELV1 Peptide Fusion scFv | TPV1 Peptide Fusion scFv | OCV1 Peptide Fusion scFv |
|---|---|---|---|---|
| Recovery Concentration | 1.17 mg/ml | 1.18 mg/ml | 1.27 mg/ml | 1.37 mg/ml |
| Recovery Efficiency | 96% | 96% | 98% | 97% |

As is clear from Table 2, every peptide fusion scFv was recovered at a high concentration, and the recovery rate was as high as 96% or more. This reveals that peptide fusion scFv can achieve refolding at high rates, and that peptide fusion scFv can be recovered highly efficiently.

2-2. Adsorption of Peptide Fusion scFv on PDMS

Fig. 4 shows the results of section 1-3. As is clear from Fig. 4, D10 peptide fusion scFv (D10-scFv) was practically not adsorbed onto the surface of the PDMS substrate. In contrast, adsorption of fusion scFv with any of ELV1 peptide, TPV1 peptide, or OCV1 peptide on the PDMS substrate increased in a concentration-dependent manner. In particular, the use of ELV1 peptide fusion scFv exhibited a significant increase in adsorption. This indicates that the use of the peptide having an affinity for PDMS makes it more efficient to immobilize a desired protein on a PDMS substrate.

2-3. Antigen-Binding Activity of Peptide Fusion scFv

Fig. 5 shows the results of section 1-4. The antigen-binding activity of peptide fusion scFv after refolding was evaluated by indirect ELISA, and as is clear from Fig. 5, every peptide fusion scFv exhibited excellent antigen-binding activity. Because the signals increased, depending on the concentration of peptide fusion scFv, peptide fusion scFv was found to have antigen-binding activity useful for antigen-antibody reaction.

2-4. Antigen-Binding Activity of Peptide Fusion scFv Immobilized

on a PDMS Substrate

Fig. 6 shows the results of section 1-5. As is clear from Fig. 6, D10 peptide fusion scFv exhibited a significantly low amount of antigen-binding. This indicates that D10 peptide fusion scFv was practically not adsorbed on the substrate. In contrast, fusion scFv with any of ELV1 peptide, TPV1 peptide, or OCV1 peptide exhibited an increased amount of antigen-binding, depending on the concentration of the antigen. This indicates that these fusion scFv have sufficient antigen-binding activity when immobilized on a PDMS substrate.

Sequence Table

PCT_polydimethylsiloxane_20160212_110714_5.txt

## Claims

1. A polydimethylsiloxane substrate to which a peptide having an affinity for polydimethylsiloxane is bound, the peptide comprising the following peptide (1a) or (1b), or a fragment thereof:

   (1a) a peptide comprising an amino acid sequence represented by at least one member selected from the group consisting of SEQ ID NOs: 1 to 9; and
   (1b) a peptide comprising the amino acid sequence of (1a) in which one or more amino acids are deleted, substituted, and/or added, the peptide having an affinity for polydimethylsiloxane.

2. The polydimethylsiloxane substrate according to claim 1, wherein the fragment comprises 15 to 58 amino acid residues.

3. The polydimethylsiloxane substrate according to claim 1 or 2, on which a target protein is immobilized via the peptide having an affinity for polydimethylsiloxane.

4. A method for immobilizing a target protein on a polydimethylsiloxane substrate, the method comprising the step of bringing a peptide having an affinity for polydimethylsiloxane into contact with the polydimethylsiloxane substrate, wherein the peptide is incorporated in the target protein, and the peptide comprises the following peptide (1a) or (1b), or a fragment thereof:

   (1a) a peptide comprising an amino acid sequence represented by at least one member selected from the group consisting of SEQ ID NOs: 1 to 9; and
   (1b) a peptide comprising the amino acid sequence of (1a) in which one or more amino acids are deleted, substituted, and/or added, the peptide having an affinity for polydimethylsiloxane.

5. A method for immobilizing a target protein on a polydimethylsiloxane substrate, the method comprising the step of binding the peptide having an affinity for polydimethylsiloxane bound to the polydimethylsiloxane substrate according to claim 1 or 2 to the target protein.

6. A composition for immobilizing a target protein on a polydimethylsiloxane substrate, the composition comprising a peptide having an affinity for polydimethylsiloxane, the peptide comprising the following peptide (1a) or (1b), or a fragment thereof:

   (1a) a peptide comprising an amino acid sequence represented by at least one member selected from the group consisting of SEQ ID NOs: 1 to 9; and
   (1b) a peptide comprising the amino acid sequence of (1a) in which one or more amino acids are deleted, substituted, and/or added, the peptide having an affinity for polydimethylsiloxane.

7. Use of a peptide having an affinity for polydimethylsiloxane in immobilizing a target protein on a polydimethylsiloxane substrate, the peptide comprising the following peptide (1a) or (1b), or a fragment thereof:

   (1a) a peptide comprising an amino acid sequence represented by at least one member selected from the group consisting of SEQ ID NOs: 1 to 9; and
   (1b) a peptide comprising the amino acid sequence of (1a) in which one or more amino acids are deleted, substituted, and/or added, the peptide having an affinity for polydimethylsiloxane.

8. A peptide having an affinity for polydimethylsiloxane, the peptide comprising the following peptide (1c) or (1d), or a fragment thereof:

   (1c) a peptide comprising an amino acid sequence represented by at least one member selected from the group consisting of SEQ ID NOs: 2 to 9; and
   (1d) a peptide comprising an amino acid sequence represented by at least one member selected from the group consisting of SEQ ID NOs: 1 to 9 in which one or more amino acids are deleted, substituted, and/or added, the peptide having an affinity for polydimethylsiloxane.

9. The peptide having an affinity for polydimethylsiloxane according to claim 8, wherein the fragment comprises 15 to 58 amino acid residues.

**10.** A polynucleotide encoding the peptide having an affinity for polydimethylsiloxane according to claim 8 or 9.

**11.** The polynucleotide according to claim 10, which is represented by at least one member selected from the group consisting of SEQ ID NOs: 10 to 18.

**12.** A vector for expressing a peptide having an affinity for polydimethylsiloxane, the vector comprising the polynucleotide according to claim 10 or 11.

**13.** A vector for expressing a peptide fusion protein,
the peptide fusion protein comprising the peptide having an affinity for polydimethylsiloxane according to claim 8 or 9 and a target protein,
the vector comprising the polynucleotide according to claim 10 or 11 and a polynucleotide encoding the target protein, wherein the polynucleotide according to claim 10 or 11 is linked to the polynucleotide encoding the target protein.

**14.** A transformant obtainable by introducing the vector according to claim 13 into a host cell to transform the host cell.

**15.** A peptide fusion protein comprising the peptide having an affinity for polydimethylsiloxane according to claim 8 or 9 and a target protein, the peptide fusion protein being obtainable from the transformant according to claim 14.

**16.** A linker for immobilizing a target protein on a polydimethylsiloxane substrate, the linker comprising the peptide having an affinity for polydimethylsiloxane according to claim 8 or 9.

Fig. 1

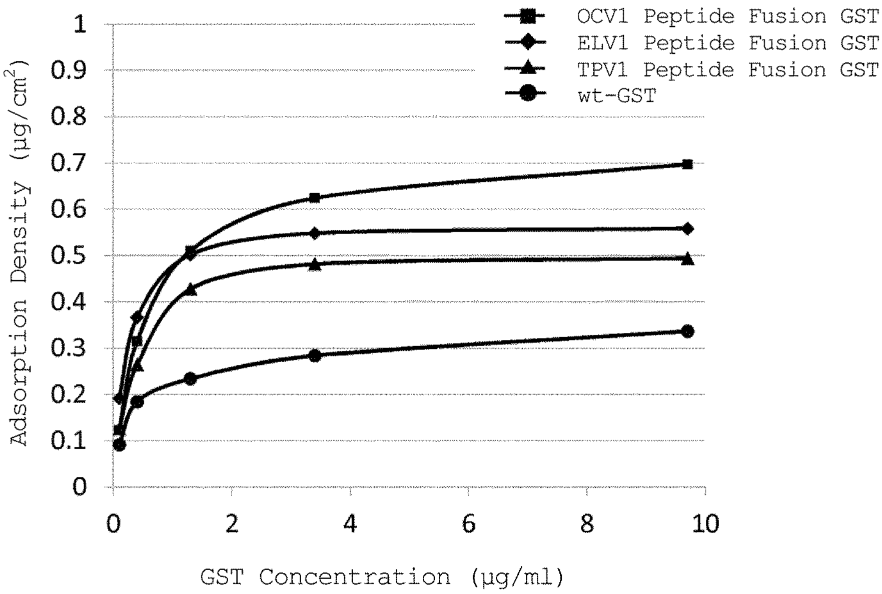


Fig. 2

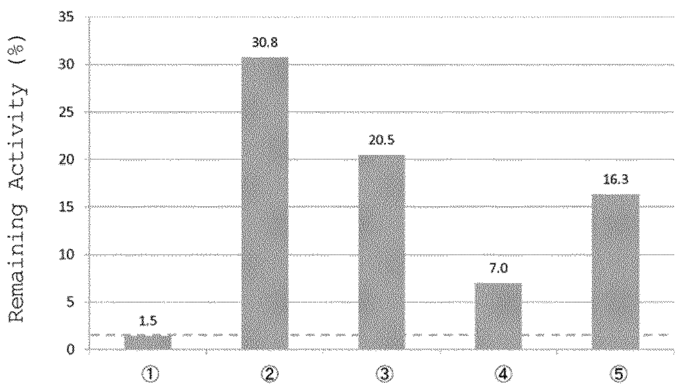


① wt-GST ② ELV1 Peptide Fusion GST ③ TPV1 Peptide Fusion GST
④ OAT1 Peptide Fusion GST ⑤ OCV1 Peptide Fusion GST

Fig. 3

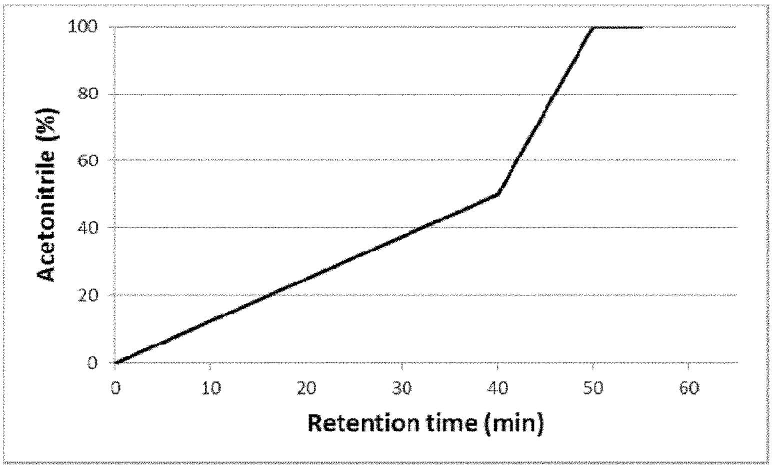
100
80
60
40
20
0
Acetonitrile (%)
0
10
20
30
40
50
60
Retention time (min)

Fig. 4

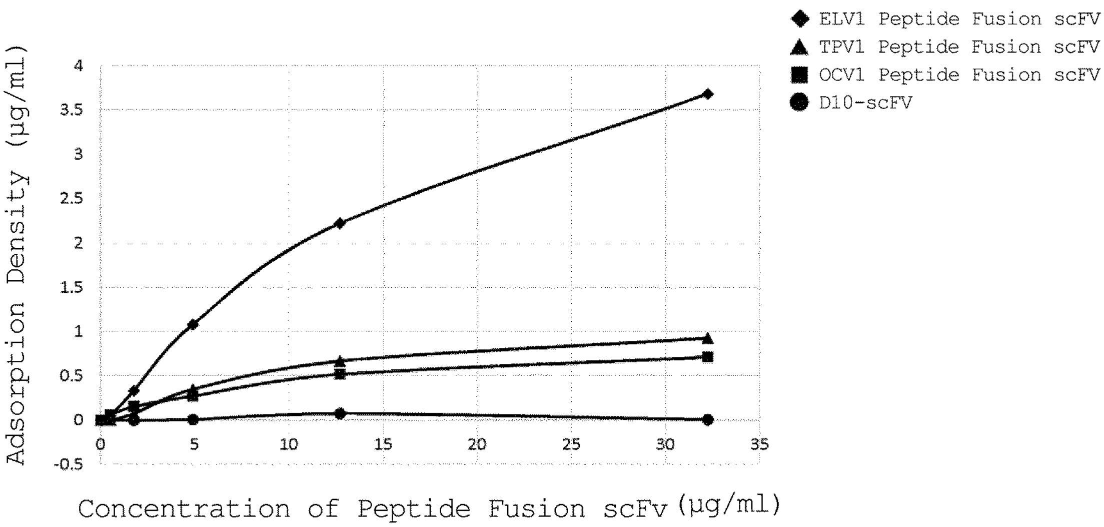
ELV1 Peptide Fusion scFV
TPV1 Peptide Fusion scFV
OCV1 Peptide Fusion scFV
D10-scFV
Adsorption Density (µg/ml)
4
3.5
3
2.5
2
1.5
1
0.5
0
-0.5
0
5
10
15
20
25
30
35
Concentration of Peptide Fusion scFv (µg/ml)

Fig. 5

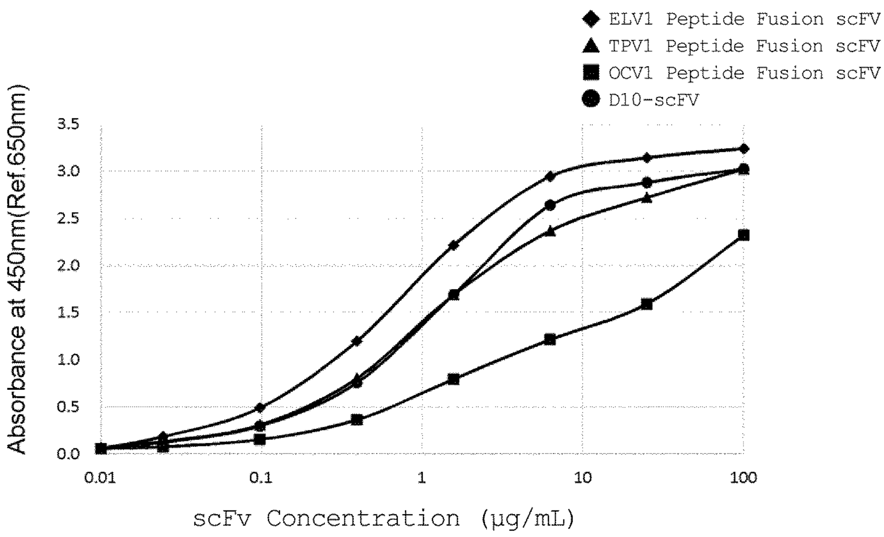
ELV1 Peptide Fusion scFV
TPV1 Peptide Fusion scFV
OCV1 Peptide Fusion scFV
D10-scFV
Absorbance at 450nm(Ref.650nm)
3.5
3.0
2.5
2.0
1.5
1.0
0.5
0.0
0.01
0.1
1
10
100
scFv Concentration (μg/mL)

Fig. 6

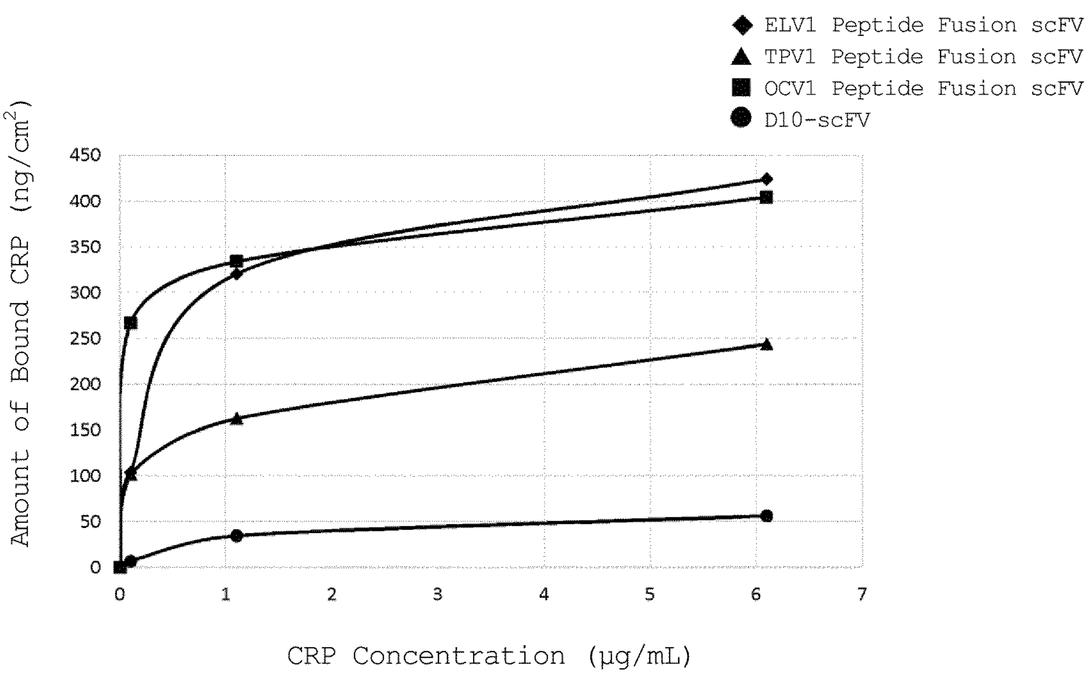
ELV1 Peptide Fusion scFV
TPV1 Peptide Fusion scFV
OCV1 Peptide Fusion scFV
D10-scFV
Amount of Bound CRP (ng/cm2)
450
400
350
300
250
200
150
100
50
0
0
1
2
3
4
5
6
7
CRP Concentration (μg/mL)

## INTERNATIONAL SEARCH REPORT

International application No.
PCT/JP2016/054195

A. CLASSIFICATION OF SUBJECT MATTER
*C07K17/08*(2006.01)i, *C07K14/00*(2006.01)i, *C12N7/08*(2006.01)i, *C12N9/00*(2006.01)i, *C12N15/00*(2006.01)i

According to International Patent Classification (IPC) or to both national classification and IPC

B. FIELDS SEARCHED

Minimum documentation searched (classification system followed by classification symbols)
C07K17/08, C07K14/00, C12N7/08, C12N9/00, C12N15/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus(JDreamIII), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 62-232395 A (Sumitomo Chemical Co., Ltd.), 12 October 1987 (12.10.1987), table 7; fig. 2 (Family: none) | 8-12 |
| X | JP 2010-516713 A (Folia Biotech Inc.), 20 May 2010 (20.05.2010), table 4 & US 2010/0316665 A1 & WO 2008/089569 A1 page 34 & CA 2676481 A & CN 101646772 A | 8-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 April 2016 (18.04.16) | 10 May 2016 (10.05.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2016/054195

C (Continuation). DOCUMENTS CONSIDERED TO BE RELEVANT

| Category* | Citation of document, with indication, where appropriate, of the relevant passages | Relevant to claim No. |
|---|---|---|
| A | WO 2009/101807 A1 (National University Corporation Kyoto Institute of Technology), 29 August 2009 (29.08.2009), entire text & JP 5553336 B & US 2011/0045538 A1 & CN 101952432 A | 1-16 |
| A | JP 2011-168505 A (National University Corporation Kyoto Institute of Technology), 01 September 2011 (01.09.2011), paragraphs [0093] to [0114] (Family: none) | 1-16 |

Form PCT/ISA/210 (continuation of second sheet) (January 2015)

INTERNATIONAL SEARCH REPORT

International application No.
PCT/JP2016/054195

Subject to be covered by this search:
With respect to claims 1-16

With taking the statement that "the range of the number of "one or multiple" in the peptide (1-2) is not particularly limited as long as the peptide can have affinity for PDMS, and the number is, for example, 1 to 15…" in paragraph [0013] into consideration, the "peptide which comprises an amino acid sequence (1b) produced by deleting, substituting and/or adding one or multiple amino acid residues in the amino acid sequence (1a) and which has affinity for polydimethylsiloxane" recited in claim 1 includes a peptide in which 15 amino acid residues are substituted. And the amino acid sequence represented by SEQ ID NO: 6 which is composed of 17 amino acid residues is mentioned as an example. Therefore, it is considered that a peptide in which almost all of amino acid residues are substituted is included within the scope of the above-mentioned peptide.

However, it cannot be considered that there is such common technical knowledge that a peptide in which many amino acid sequences are substituted has the same properties as those of the original peptide thereof, and a base on which the contents disclosed in the description can be expanded or generalized to the scope of the invention described in claim 1 cannot be found.

Consequently, the invention of claim 1 exceeds the range set forth in the description, and does not comply with the requirement of the support prescribed under PCT Article 6.

In addition, even with taking the above-mentioned statements in the description and the common technical knowledge at the time of filing the present application into consideration, it is impossible to understand as to what types of peptides the above-mentioned "peptide which comprises an amino acid sequence (1b) produced by deleting, substituting and/or adding one or multiple amino acid residues in the amino acid sequence (1a) and which has affinity for polydimethylsiloxane" means specifically. Therefore, it is considered that, in the practice of the invention described in claim 1, it is required to make a trial-and-error procedure that beyonds the level that can be expected of persons skilled in the art, such as a procedure of producing an infinite number of peptides and screening for the peptide from the peptides and confirming the screened peptide.

Consequently, the description is not clearly and fully set forth to such a degree that a person skilled in the art can perform the invention of claim 1, and therefore does not comply with the requirement prescribed under PCT Article 5.

Furthermore, it is obvious that the "peptide which comprises an amino acid sequence (1b) produced by deleting, substituting and/or adding one or multiple amino acid residues in the amino acid sequence (1a) and which has affinity for polydimethylsiloxane" is not specified technically sufficiently, and therefore it is impossible to clearly understand the invention from the statements in claim 1 even though the statements in the description are taken in consideration.

Therefore, claim 1 does not comply with also the requirement of clarity prescribed under PCT Article 6.

Further, also the invention of claims 2-16 do not comply with the requirements prescribed under PCT Article 5 and Article 6 for the same reason.

Such being the case, the search on parts of the inventions described in claims 1-16 which do not relate to peptides respectively comprising the amino acid sequences represented by SEQ ID NOs: 1 to 9 which are specifically mentioned was not carried out, because it is impossible to carry out a meaningful search on the parts.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009101807 A1
- JP 2011168505 A


### Non-patent literature cited in the description


- **SAMBROOK et al.** Molecular Cloning: A laboratory manual. Cold Spring Harbor Laboratory Press, 1989
- *Proc. Natl. Acad. Sci.,* 1981, vol. 78, 6613
- *Science,* 1983, vol. 222, 778
- Molecular Cloning. Cold Spring Harbor Lab. Press, 1989
- Lectures on Biochemical Experiments (Genetic Research Methods I, II, III). *Journal of The Japanese Biochemistry Society,* 1986
- Biochemistry Data Book II. Kagaku Dojin Co., Inc, 1980, 1175-1259
- *Biochemistry,* 1986, vol. 25 (25), 8274
- *Eur. J. Biochem.,* 1987, vol. 163, 313